# EUROPÄISCHE PATENTSCHRIFT

(11) **EP 1 087 733 B1**
(45) Veröffentlichungstag und Bekanntmachung des Hinweises auf die Patenterteilung: **31.07.2002**
(21) Anmeldenummer: 99920837.4
(22) Anmeldetag: 03.05.1999
(51) Int. Cl.: A61F 2/78

(54) **ADAPTER FÜR EIN EXOPROTHETISCHES STANDARDTEIL**
ADAPTER FOR AN EXOPROSTHETIC STANDARD ELEMENT
ADAPTATEUR POUR UN ELEMENT STANDARD D'EXOPROTHESE

(30) Priorität: 17.06.1998 DE 19826638
(43) Veröffentlichungstag der Anmeldung: 04.04.2001
(73) Patentinhaber: ESKA Implants GmbH & Co., 23556 Lübeck (DE)
(72) Erfinder: GRUNDEI, Hans, D-23558 Lübeck (DE)
(74) Vertreter: Fuchs Mehler Weiss & Fritzsche
(86) Internationale Anmeldenummer: EP9902988
(87) Internationale Veröffentlichungsnummer: WO9965426

(56) Entgegenhaltungen:
- DE-C- 4 106 971
- US-A- 3 947 897
- US-A- 4 158 895
- US-A- 5 041 137
- US-A- 5 759 206

## Beschreibung

Die Erfindung betrifft einen Adapter für ein exoprothetisches Standardteil.

Bei der exoprothetischen Versorgung eines gliedmaßenamputierten Patienten gibt es unterschiedliche Wege, die bereits beschritten werden.

So ist es denkbar, ausschließlich mit exoprothetischen, beispielsweise Prothesenschäften zu arbeiten beispielsweise im Bereich eines Oberschenkelstumpfes, die an die Größe und Formen des Oberschenkelstumpfes angepaßt werden. Mit diesem Prothesenschaft sind dann ggfls. ein künstliches exoprothetisches Kniegelenk, ein Unterschenkel sowie Fuß verbunden.

Darüberhinaus gibt es jedoch Systeme, die mit den verbliebenen Röhrenknochenstumpfenden verankert werden, so daß dem Patienten ein natürlicheres Wahrnehmungsvermögen, beispielsweise beim Laufen, vermittelt werden kann. Letzteres ist bekannt unter der Bezeichnung Osteoperzeption.

Hierzu wird beispielsweise ein Adapterende mit einem Gewinde in den verbliebenen Röhrenknochen geschraubt, um so mit diesem verankert zu werden. Der Adapter ähnelt einem Rohrstück, der am Stumpfende schließlich aus dem Gewebe heraustritt. Daran anzukoppeln ist dann ein exoprothetisches Standardteil, beispielsweise ein künstliches Kniegelenk.

Bei den bekannten Adaptern der vorbezeichneten Art ist die Stabilität der Fixation des Adapters im Röhrenknochenstumpf problematisch, ebenso wie die Ankopplung eines Standardteiles.
Aus der US-A-3,947,897 ist ein Adapter bekannt, der in den intermedullären Raum des Femurs gesetzt wird, der an seinem distalen Ende. mit einer Kopplungseinrichtung für ein exoprothetisches Standardteil versehen ist. Der Sitz im intramedullären Raum des Femurs soll fixiert werden durch Anwendung eines Knochenzementes, beispielsweise Methylmethacrylat. Die Ankopplung des Standardteiles am distalen Ende soll durch eine Art Verriegelungsvorrichtung bewerkstelligt werden.
Sowohl die Fixierung mit Knochenzement als auch die Fixierung des Standardteiles mit der Verriegelungsvorrichtung ist problematisch.
Knochenzement lockert sich nach einer gewissen Zeit. Auch die Verriegelungsvorrichtung hält einem dauerhaften Sitz des exoprothetischen Standardteils nicht stand.
Vor diesem Hintergrund ist es die Aufgabe der vorliegenden Erfindung, einen Adapter für ein exoprothetisches Standardteil anzugeben, welches ein günstiges Langzeitfixationsverhalten aufweist und es darüberhinaus gestattet, leicht ein Standardteil gegen ein anderes, beispielsweise nach fortgeschrittenem Abrieb, auszutauschen.
Gelöst wird diese Aufgabe durch einen Adapter mit den Merkmalen des Anspruchs 1.
Demnach wird vorgeschlagen, den Adapter so auszubilden, daß er mit einem proximalen Stielteil in einen Röhrenknochenstumpf setzbar ist, wobei das Stielteil zumindest teilweise mit einer offenmaschigen, dreidimensionalen Raumnetzstrukur bedeckt ist und an seinem anderen, distalen Ende mit einer Koppelungseinrichtung für ein exoprothetisches Standardteil versehen ist und wobei die Kopplungseinrichtung aus einem Konus und einer darauf im konischen Klemmsitz sitzenden Adapterhülse besteht, auf der ein Kuppelungsteil sitzt, mit dem das exoprothetische Standardteil verspannbar ist.
Die offenmaschige, dreidimensionale Raumnetzstruktur, die auch als interkonnektierend bezeichnet wird, ermöglicht ein Ein-, Durch-, Hinter- und Umwachsen von natürlichem Knochenmaterial während der Einheilphase, so daß der Stielteil nach relativ kurzer Zeit jedenfalls im Hinblick auf den Substratfluß im Röhrenknochen integriert wird und eine extrem stabile Sekundärfixation gewährleistet wird. Das Einwachsen von Knochenmaterial in eine offenmaschige, dreidimensionale Raumnetzstruktur ist an sich bekannt aus der reinen Endoprothetik. Hier sei beispielsweise auf die DE-PS 41 06 971 hingewiesen. Vorliegend aber kommt eine derartige Fixationsmöglichkeit erstmalig im Bereich der exoprothetischen Versorgung zum Einsatz. Die distalseitig vorgesehene Koppelungseinrichtung ist freilich ausschließlich im Zusammenhang mit der hervorragenden Sekundärfixation aufgrund der genannten Raumnetzstruktur zu sehen. Die Verbleibdauer nämlich des Adapters im Röhrenknochenstumpf ist so lange, daß externe Abriebs- oder Ausschlagserscheinungen auftreten können, die einen exakten Sitz des exoprothetischen Standardteils an der Ankoppelungsstelle nicht mehr gewährleisten könnten.

Dies Adapterhülse hat den Vorteil, austauschbar zu sein, etwa wenn die Ankopplung eines Standardteils im Laufe der Zeit zum vermehrten Abrieb oder Ausschlagungen geführt hat. Die Adapterhülse als solches kann gesamthaft ausgetauscht werden.

Der Sitz der Adapterhülse auf dem Konus der Koppelungseinrichtung kann auch zusätzlich dadurch gesichert sein, daß eine Spannschraube durch die Adapterhülse hindurch in eine Gewindebohrung im Konus geschraubt ist.

Es ist gemäß einer weiteren vorteilhaften Weiterbildung vorgesehen, das Stielteil mit einem umlaufenden Flansch zur Anlage an der Stirnkante des Röhrenknochenstumpfes zu versehen. Hierdurch wird der Adapter direkt nach der Implantation des Stielteils im Röhrenknochenstumpf belastbar, bevor nämlich genügend Knochentrapekel in die offenmaschige dreidimensionale Raumnetzstruktur des Stielteils gewachsen sind. Der Flansch dient also zur primären Ablastung der auftretenden Kräfte während der Einheilphase und der Einleitung der auftretenden Kräfte in die Kortikalis des Röhrenknochenstumpfes.

Bevorzugt schließt sich dem Stielteil in Richtung der distalen Koppelungseinrichtung ein Zwischenstück an, welches dann vom Röhrenknochenstumpfende weiter durchs Körpergewebe verläuft, bis die Koppelungseinrichtung extrakorporal liegt. An der Durchtrittsstelle des Zwischenstückes aus dem Patientenkörper sitzt dann vorzugsweise ein Abdichtteil auf dem Zwischenstück. Dieses Abdichtteil schützt die Durchbruchstelle im Gliedmaßenstumpf und trägt dazu bei, daß sich die Durchbruchstelle leichter aseptisch halten lassen kann. Schweren Entzündungen, die sich in den Gliedmaßenstumpf hinein fortentwickeln könnten, wird so eine Hürde gebaut.

Das Zwischenstück des Adapters kann gemäß einer Ausführungsform in seiner Länge einstellbar sein. Dies hat den Vorteil, daß lediglich ein oder wenige Konfektionsgrößen des erfindungsgemäßen Adapters auf Lager gehalten werden müssen. Dies reduziert die Kosten erheblich.

Alternativ besteht das Zwischenstück aus massivem Metall, wodurch die Stabilität erhöht wird. Allerdings sind hier Längseinstellungen nicht möglich, so daß mit erhöhten Kosten zu rechnen wäre.

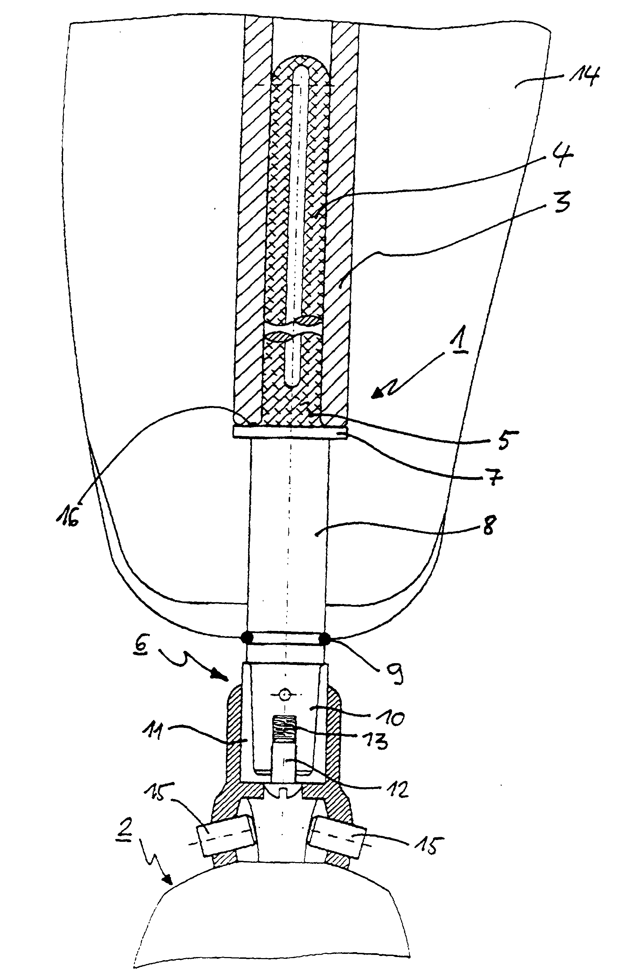

Die Erfindung wird anhand eines Ausführungsbeispiels gemäß der einzigen Zeichnungsfigur näher erläutert.

Diese zeigt einen schematischen Schnitt eines Oberschenkelstumpfes 14 mit dem Femurstumpf 3. In den röhrenförmigen Femurstumpf 3 eingeführt ist das Stielteil 4 des Adapters 1. Die Oberfläche des Stielteils 4 ist vorliegend mit einer offenmaschigen, dreidimensionalen Raumnetzstruktur 5 belegt, durch welche hindurch Knochentrapekel wachsen können, so daß das Stielteil 4 nach einer gewissen Einheilphase, was den Substratfluß betrifft, quasi Bestandteil des natürlichen Knochens geworden ist.

Das Stielteil 4 ist distalseitig abgeschlossen mit einem umlaufenden Flansch 7, der an der Stirnkante 16 des Röhrenknochenstumpfes 3 anschlägt. Dies gewährleistet zum einen eine primäre Belastbarkeit des Adapters 1 direkt nach der Implantation, und zum anderen ein Einleiten auftretender Belastungen zumindest teilweise direkt in die Kortikalis hinein.

Dem Stielteil 4 bzw. Flansch 7 schließt sich ein Zwischenstück 8 an, das vorliegend einstöckig aus massivem Metall besteht. Das Zwischenstück 8 weist eine solche Dimension auf, daß es aus dem Gliedmaßenstumpf 14 distalseitig austreten kann. Die Austrittsstelle ist vorliegend mit einem umlaufenden Abdichtteil 9 auf dem Zwischenstück 8 abgeschlossen.

Das Zwischenstück 8 endet in einem Konus 10 der Koppelungseinrichtung 6. Auf dem Konus 10 sitzt in konischer Verklemmung eine Adapterhülse 11, deren konischer Klemmsitz auf dem Konus 10 zusätzlich gesichert ist durch eine Spannschraube 12, die durch die Adapterhülse 11 in eine Gewindebohrung 13 im Konus 10 geschraubt ist.

Über die Adapterhülse 11 ist das Kupplungsteil 16 gestülpt, mit dem das exoprothetisches Standardteil 2 durch Spannschrauben 15 verbunden ist.

Kommt es im Laufe der Tragzeit zu Abriebserscheinungen oder schlagen Teile der Hülsen aus, so kann die Adapterhülse 11 leicht dadurch ausgetauscht werden, daß die Spannschraube 12 aus der Gewindebohrung 13 geschraubt wird, die alte Adapterhülse 11 vom Konus 10 abgezogen wird und durch eine neue Adapterhülse 11 ersetzt wird. Sodann kann wieder das exoprothetische Standardteil montiert werden.

## Patentansprüche

1. Adapter (1) für ein exoprothetisches Standardteil (2), der mit einem proximalen Stielteil (4) in einen Röhrenknochenstumpf (3) setzbar ist, wobei das Stielteil (4) zumindest teilweise mit einer offenmaschigen, dreidimensionalen Raumnetzstruktur (5) bedeckt ist und an seinem distalen Ende mit einer Koppelungseinrichtung (6) für das exoprothetische Standardteil (2) versehen ist, bei dem die Koppelungseinrichtung (6) aus einem Konus (10) und einer darauf im konischen Klemmsitz sitzenden Adapterhülse (11) besteht, auf der ein Kupplungsteil (16) sitzt, mit dem das exoprothetische Standardteil (2) verspannbar ist.

2. Adapter nach Anspruch 1, bei dem der konische Klemmsitz der Adapterhülse (11) auf dem Konus (10) zusätzlich gesichert ist durch eine Spannschraube (12), die durch die Adapterhülse (11) hindurch in eine Gewindebohrung (13) im Konus (10) geschraubt ist.

3. Adapter nach Anspruch 1 oder 2, bei dem das Stielteil mit einem umlaufenden Flansch (7) zur Anlage an der Stirnkante (16) des Röhrenknochenstumpfes (3) versehen ist.

4. Adapter nach einem der Ansprüche 1 bis 3, bei dem sich dem Stielteil (2) in Richtung auf die Koppelungseinrichtung (6) ein Zwischenstück (8) anschließt.

5. Adapter nach Anspruch 4, bei dem an der Durchtrittsstelle des Zwischenstückes (8) aus dem Körper ein Abdichtteil (9) auf dem Zwischenstück (8) sitzt.

6. Adapter nach einem der Ansprüche 4 oder 5, bei dem das Zwischenstück (8) in seiner Länge einstellbar ist.

7. Adapter nach einem der Ansprüche 4 bis 6, bei dem das Zwischenstück (8) aus massivem Metall besteht.

## Claims

1. Adapter (1) for an exoprosthetic standard part (2), which can be seated with a proximal stem part (4) in a tubular bone stump (3), wherein the stem part (4) is covered at least partly by an open-meshed, three-dimensional spatial network structure (5) and is provided at its distal end with a coupling device (6) for the exoprosthetic standard part (2), in which the coupling device (6) consists of a cone (10) and an adapter sleeve (11) sitting thereon in the conical press fit, on which adapter sleeve (11) a coupling part (16) sits, with which the exoprosthetic standard part (2) can be braced.

2. Adapter according to claim 1, in which the conical press fit of the adapter sleeve (11) on the cone (10) is additionally secured by a tightening screw (12), which is screwed through the adapter sleeve (11) into a threaded bore (13) in the cone (10).

3. Adapter according to claim I or 2, in which the stem part is provided with an annular flange (7) for resting against the end-face edge (16) of the tubular bone stump (3).

4. Adapter according to one of claims 1 to 3, in which an intermediate piece (8) is connected to the stem part (2) in the direction of the coupling device (6).

5. Adapter according to claim 4, in which a sealing part (9) sits on the intermediate piece (8) at the passage point of the intermediate piece (8) from the body.

6. Adapter according to one of claims 4 or 5, in which the length of the intermediate piece (8) can be adjusted.

7. Adapter according to one of claims 4 to 6, in which the intermediate piece (8) consists of solid metal.

## Revendications

1. Adaptateur (1) pour une pièce standard d'exoprothèse (2), qui peut être posé avec l'extrémité proximale d'une tige(4) dans un os long tronqué (3), la tige (4) étant revêtue au moins en partie par une structure réticulée tridimensionnelle (5) à mailles ouvertes, et qui est muni sur son extrémité distale d'un dispositif de couplage (6) destiné à la pièce standard d'exoprothèse (2), dans lequel adaptateur le dispositif de couplage (6) est formé par un cône (10) et une gaine d'adaptateur (11), positionnée sur celui-ci par un ajustement conique bloqué et sur laquelle est posée une pièce de couplage (6), avec laquelle la pièce standard d'exoprothèse (2) peut être assemblée.

2. Adaptateur selon la revendication 1, dans lequel le positionnement par ajustement conique bloqué de la gaine d'adaptateur (11) sur le cône (10) est sécurisé en plus par une vis de serrage (12), qui passe à travers la gaine d'adaptateur (11) dans une forure filetée (13) réalisée dans le cône (10).

3. Adaptateur selon la revendication 1 ou 2, dans lequel la tige est munie d'une collerette (7) périphérique destinée à venir en appui contre le bord frontal (16) de l'os long tronqué (3).

4. Adaptateur selon l'une quelconque des revendications 1 à 3, dans lequel la tige (4) se prolonge en direction du dispositif de couplage (6) par une pièce intermédiaire (8).

5. Adaptateur selon la revendication 4, dans lequel une pièce d'étanchéité (9) est posée sur la pièce intermédiaire (8) dans la zone dans laquelle la pièce intermédiaire (8) s'avance hors du corps.

6. Adaptateur selon la revendication 4 ou 5, dans lequel la pièce intermédiaire (8) est réglable en longueur.

7. Adaptateur selon l'une quelconque des revendications 4 à 6, dans lequel la pièce intermédiaire (8) est réalisée en métal massif.
